# EUROPÄISCHE PATENTSCHRIFT

(11) **EP 3 496 638 B1**
(45) Veröffentlichungstag und Bekanntmachung des Hinweises auf die Patenterteilung: **05.10.2022**
(21) Anmeldenummer: 17748796.4
(22) Anmeldetag: 07.08.2017
(51) Int. Cl.: A61B 18/12

(54) **ELEKTROCHIRURGIESYSTEM MIT MESSEINHEIT**
ELECTROSURGICAL SYSTEM COMPRISING A MEASURING UNIT
SYSTÈME D'ÉLECTROCHIRURGIE DOTÉ D'UNE UNITÉ DE MESURE

(30) Priorität: 08.08.2016 DE 102016214704
(43) Veröffentlichungstag der Anmeldung: 19.06.2019
(73) Patentinhaber: Olympus Winter & Ibe GmbH, 22045 Hamburg (DE)
(72) Erfinder: FÄHSING, Thomas, 38889 Blankenburg (DE)
(74) Vertreter: Eisenführ Speiser
(86) Internationale Anmeldenummer: PCT/EP2017/069968
(87) Internationale Veröffentlichungsnummer: WO 2018/029154

(56) Entgegenhaltungen:
- EP-A1- 2 540 243
- DE-A1- 10 224 154
- US-A1- 2014 074 084
- US-A1- 2014 364 843

## Beschreibung

Die Erfindung betrifft ein Elektrochirurgiesystem zum Behandeln eines Gewebes. Weiterhin betrifft die Erfindung einen Elektrochirurgiegenerator zum Behandeln eines Gewebes.

Es ist bekannt, Gewebe mittels hochfrequenter Wechselspannung zu behandeln, beispielsweise zu schneiden oder zu koagulieren. Während einer Behandlung von Körpergewebe im Rahmen der Hochfrequenz-Chirurgie (HF-Chirurgie), kann eine Impedanz des zu behandelnden Gewebes bestimmt werden um beispielweise einen Grad der Austrocknung des Gewebes zu erfahren. Ziel ist es hierbei, die Abgabe einer zur Behandlung des Körpergewebes geeigneten Behandlungs-Wechselspannung abzubrechen, sobald die Impedanz des Gewebes ein bestimmtes Maß erreicht hat, um beispielsweise ungewollte Carbonisierung von Gewebe oder auch das Anhaften von Gewebe an Elektroden eines entsprechenden HF-Instruments zu vermeiden.

Zu diesem Zweck sind Elektrochirurgiesysteme bekannt, die dazu in der Lage sind, die Abgabe von Behandlungs-Wechselspannung in Abhängigkeit von der erfassten Impedanz des zu behandelnden Gewebes zu steuern. US2014074084 offenbart ein solches System als Stand der Technik.

Der Erfindung liegt die Aufgabe zugrunde, ein verbessertes Elektrochirurgiesystem zum Behandeln eines Gewebes bereitzustellen.

Erfindungsgemäß wird diese Aufgabe durch ein Elektrochirurgiesystem gelöst, dass eine erste Elektrode und eine zweite Elektrode, eine Hochfrequenz-Hochspannungsversorgungseinheit, eine Messeinheit und eine Steuereinheit aufweist.

Die erste und zweite Elektrode sind dabei jeweils ausgebildet, leitend mit dem zu behandelnden Gewebe verbunden zu sein.

Die Hochfrequenz-Hochspannungsversorgungseinheit ist mit der ersten Elektrode über eine erste elektrische Leitung und mit der zweiten Elektrode über eine zweite elektrische Leitung elektrisch verbindbar und ausgebildet, diese Elektroden mit einer vorbestimmten Behandlungs-Wechselspannung zu versorgen.

Die Messeinheit ist mit der ersten und zweiten Elektrode elektrisch verbindbar und weist ein Messspannungsversorgungsmodul auf, welches ausgebildet ist, MessWechselspannung mindestens einer ersten und einer zweiten Frequenz bereitzustellen, wobei die Messeinheit weiterhin ausgebildet ist, über die erste und zweite Elektrode ein elektrisches Antwortsignal für die erste und zweite Frequenz aufzunehmen und daraus mindestens eine Eigenschaft eines zwischen der ersten und zweiten Elektrode befindlichen Gewebes zu bestimmen.

Die Steuereinheit ist ausgebildet, während eines Betriebs des Elektrochirurgiesystems zu einem Messbeginn die elektrische Verbindung der Hochfrequenz-Hochspannungsversorgungseinheit mit der ersten oder zweiten Elektrode zu unterbrechen und die elektrische Verbindung der Messeinheit mit der ersten und zweiten Elektrode herzustellen. Weiterhin ist die Steuereinheit ausgebildet, zu einem Messabschluss die elektrische Verbindung der Messeinheit mit der ersten oder zweiten Elektrode zu unterbrechen und die elektrische Verbindung der Hochfrequenz-Hochspannungsversorgungseinheit mit der ersten und zweiten Elektrode herzustellen.

Die Erfindung schließt die Erkenntnis ein, dass elektrische Parameter des zu behandelnden Gewebes herkömmlich nur bei der Arbeitsfrequenz der Hochfrequenz-Hochspannungsversorgungseinheit ermittelt werden können. Hierdurch gehen Informationen bezüglich Eigenschaften des Gewebes bei Nutzung einer einzigen Arbeitsfrequenz verloren oder können zumindest nur unzureichend erfasst werden.

Daher ist es vorteilhaft, zusätzlich zu einer Behandlungs-Wechselspannung der Hochfrequenz-Hochspannungsversorgungseinheit eine separate Messeinheit mit einer MessWechselspannung einer ersten und zweiten Frequenz zu nutzen. Hierdurch können mehr Informationen über das zu behandelnde Gewebe bestimmt werden, was im Sinne einer kontrollierten Behandlung vorteilhaft ist. Informationen über das zu behandelnde Gewebe können beispielsweise eine Information zu einem vorliegenden Gewebezustand, wie etwa einem Grad an Austrocknung des Gewebes, oder zu einem vorliegenden Gewebetyp sein.

Weiterhin ist vorteilhaft, dass das erfindungsgemäße Elektrochirurgiesystem von einem Behandlungszustand mit an dem Gewebe anliegender Behandlungs-Wechselspannung zu einem Messzustand mit an dem Gewebe anliegender Mess-Wechselspannung umschalten kann und umgekehrt auch wieder zurück zu dem Behandlungszustand wechseln kann, entsprechend einer Steuerung durch die Steuereinheit. Durch diese klare Trennung zwischen den beiden Zuständen des Elektrochirurgiesystems lässt sich deutlich abgrenzen, wann die Eigenschaften des Gewebes bestimmt werden und wann das Gewebe behandelt wird. Beide Prozesse können präzise durchgeführt werden, ohne sich einander zu beeinflussen.

Eine erfindungsgemäße Nutzung einer über die Messeinheit gesteuerten separaten Messschaltung verbessert insbesondere eine Sicherheit des Elektrochirurgiesystems, da die Steuerung der Messeinheit keinen unmittelbaren Einfluss auf einen Betrieb der Hochfrequenz-Hochspannungsversorgungseinheit hat. Hierdurch kann die Behandlung des Gewebes allein durch Eigenschaften der Hochfrequenz-Hochspannungsversorgungseinheit festgelegt sein, ohne dass elektrische Widerstände der Messeinheit Einfluss auf die Behandlung haben.

Weiterhin kann das erfindungsgemäße Elektrochirurgiesystem mit bekannten Elektroden verwendet werden, da die Messeinheit und die Steuereinheit Teile eines die Hochfrequenz-Hochspannungsversorgungseinheit umfassenden Elektrochirurgiegenerators sein könnten. Hierdurch ist das erfindungsgemäße Elektrochirurgiesystem besonders einfach in bestehende HF-Chirurgiesysteme integrierbar.

Die Nutzung variabler Behandlungspausen zwischen Messbeginn und Messabschluss führt zu einer zeitlichen Trennung zwischen dem Messen von Eigenschaften des Gewebes und dem Behandeln dieses Gewebes. Während die Arbeitsfrequenz der Hochfrequenz-Hochspannungsversorgungseinheit in einem Bereich zwischen 350 und 500 kHz liegt und über einen Behandlungszeitraum üblicherweise konstant ist, kann die MessWechselspannung der Messeinheit zeitlich variiert werden und in einem für die Messung der Eigenschaften des Gewebes besonders geeigneten Frequenzbereich liegen.

Der Messbeginn wird durch eine externe oder interne Steuerung ausgelöst, und stellt den Beginn eines von der Messeinheit und der Steuereinheit ausgeführten Verfahrens zur Messung von Eigenschaften eines zu behandelnden Gewebes dar. Hierbei erfolgt eine Messung durch die Messeinheit vorzugsweise separat zu einer Steuerung der vorliegenden elektrischen Verbindungen durch die Steuereinheit.

Die erste und zweite Elektrode können in einem gemeinsamen elektrochirurgischen Instrument angeordnet sein. Weiterhin kann es sich bei der ersten oder zweiten Elektrode um eine aktive Elektrode handeln, während die jeweils andere zweite oder erste Elektrode eine passive Elektrode ist. Eine genaue Ausprägung der Elektroden ist für die vorliegende Erfindung nicht wesentlich, insbesondere ist das erfindungsgemäße Elektrochirurgiesystem mit allen gängigen Elektrodenarten, bei denen die erste und zweite Elektrode leitend mit dem zu behandelnden Gewebe verbunden sind, kombinierbar.

Im Folgenden seien bevorzugte Ausführungsformen des erfindungsgemäßen Elektrochirurgiesystems beschrieben.

In einer besonders bevorzugten Ausführungsform ist die Messeinheit ausgebildet, die Impedanz des zwischen der ersten und zweiten Elektrode befindlichen Gewebes jeweils für die Mess-Wechselspannung mit der ersten und zweiten Frequenz zu bestimmen. Die Bestimmung der Impedanz ist dadurch besonders vorteilhaft, dass daraus auf dielektrische Eigenschaften des behandelten Gewebes geschlossen werden kann. Hierdurch kann beispielsweise ein Anteil von Wasser in dem Gewebe bestimmt werden. Für die Bestimmung der Impedanz genügt es neben der Frequenz die Mess-Wechselspannung und eine Stärke des Mess-Stroms zu bestimmen. Dies kann über bekannte Messgeräteanordnungen realisiert sein.

In einer bevorzugten Variante der vorherigen Ausführungsform ist die Messeinheit weiterhin ausgebildet, eine Differenz der bei mindestens zwei unterschiedlichen Frequenzen gemessenen Impedanz des Gewebes als Relativparameter zu ermitteln. Hierbei ist in einem Beispiel der Variante die Differenz der Impedanz die Differenz zwischen den Impedanzbeträgen. Die jeweils gemessenen Impedanzwerte können durch Messwerte repräsentiert werden die zweidimensional sind, also einen Realteil und einen Imaginärteil haben. In einem weiteren Beispiel ist die Differenz der Impedanz die Differenz zwischen den Beträgen der Realteile der gemessenen Impedanzen. Mit zunehmender Koagulation des Gewebes wird die Differenz kleiner werden, so dass ein Grad der Koagulation die ermittelte Eigenschaft des Gewebes ist.

Erfindungsgemäß ist die Steuereinheit mit der Messeinheit elektrisch verbunden und weiterhin ausgebildet, nach dem Messbeginn eine Aktivierung der MessWechselspannung mit der ersten Frequenz durch das Messspannungsversorgungsmodul auszulösen und vor dem Messabschluss eine Deaktivierung einer MessWechselspannung des Messspannungsversorgungsmoduls auszulösen. Es wird in dieser Ausführungsform erst dann eine Messung mit einer Messwechselspannung begonnen, wenn die Messeinheit mit dem Gewebe elektrisch verbunden ist und es wird erst dann die Verbindung unterbrochen, wenn die Messung über die Mess-Wechselspannung abgeschlossen ist. Hierdurch wird die Gefahr von Spannungsspitzen an durch die Steuereinheit betätigten Schaltern reduziert und ein kontrollierter, zumindest vorläufiger Abschluss des gewünschten Messprozesses sichergestellt.

Das Bestimmen der Eigenschaft des zwischen den beiden Elektroden befindlichen Gewebes erfolgt vorzugsweise in einem Signalverarbeitungsmodul der Messeinheit, welches die elektrischen Antwortsignale für die erste und zweite Frequenz empfängt. In einer Variante ist das Signalverarbeitungsmodul der Messeinheit weiterhin mit einem Speichermodul der Messeinheit verbunden und ausgebildet, die Eigenschaft des Gewebes auf Basis eines Vergleichs mit einem in dem Speichermodul gespeicherten Wert zu bestimmen.

Vorzugsweise werden im Rahmen eines durch die Messeinheit durchgeführten Messprozesses Mess-Wechselspannungen mit mehr als zwei verschiedenen Frequenzen verwendet. Vorzugsweise wird ein Antwortsignal für eine Vielzahl von Frequenzen eines Frequenzbereichs bestimmt. Beispielsweise kann die erste Frequenz im Frequenzband zwischen 10 kHz und 50 kHz liegen, die zweite Frequenz im Frequenzband zwischen 200 kHz und 600 kHz und eine dritte Frequenz in einem Frequenzband zwischen 800 kHz und 1,2 MHz. In einer Variante dieser Ausführungsform wird entsprechend der Vielzahl von Frequenzen auch eine Vielzahl von Differenzen gemessener Impedanzen des Gewebes bestimmt.

In einer weiteren Ausführungsform ist eine Zeitdauer zwischen Messbeginn und Messabschluss eine vordefinierte Messdauer ist. In dieser Ausführungsform bedarf das Auslösen des Messabschlusses keiner separaten internen oder externen Eingabe, da mit einem Auslösen des Messbeginns bereits ein Zeitpunkt des Messabschlusses festgelegt ist.

In einer alternativen Ausführungsform ist die Messdauer zwischen dem Messbeginn und dem Messabschluss nicht vordefiniert. In dieser Ausführungsform wird der Messprozess der Messeinheit so lange fortgeführt, bis die Messeinheit ein Signal empfängt, das den Messabschluss durch die Messeinheit und die Steuereinheit auslöst.

In einer Ausführungsform ist das Elektrochirurgiesystem derart konfiguriert, dass der Messbeginn automatisiert in bestimmten, insbesondere vordefinierten Zeitabständen von der Steuereinheit ausgelöst wird. In dieser Ausführungsform ist kein weiteres Signal, beispielsweise durch Nutzereingabe, zum Auslösen des Messbeginns erforderlich. Vorteilhaft kann hierdurch ein regelmäßiges Messen der Eigenschaft des Gewebes sichergestellt werden ohne dafür eine weitere Betriebseinheit oder eine manuelle Nutzereingabe zu benötigen. Dies reduziert das Risiko eines zu seltenen Bestimmens der Eigenschaft des Gewebes während eines Betriebs des erfindungsgemäßen Elektrochirurgiesystems. Weiterhin wird durch das automatisierte Auslösen des Messbeginns eine Automatisierung der gesamten Behandlung des Gewebes durch das Elektrochirurgiesystem vereinfacht.

Vorzugsweise wird im Rahmen des erfindungsgemäßen Elektrochirurgiesystems ein regelmäßiger Wechsel zwischen einem Behandlungszustand, in dem die Behandlungs-Wechselspannung an dem Gewebe angelegt ist, und einem Messzustand, in dem die Messeinheit das Gewebe mit der Mess-Wechselspannung versorgt, vorliegen.

In einer weiteren Ausführungsform ist das Elektrochirurgiesystem ausgebildet, den Messbeginn manuellen auszulösen, wobei eine Nutzerschnittstelle ausgebildet ist, ein entsprechendes Auslösesignal im Falle einer Nutzereingabe für die Steuereinheit bereitzustellen. Die Nutzerschnittstelle kann beispielsweise ein Knopf sein, den ein Nutzer des Elektrochirurgiesystems in regelmäßigen Zeitabständen drückt.

In einer besonders bevorzugten Ausführungsform ist die Steuereinheit ausgebildet, die elektrische Verbindung der Hochfrequenz-Hochspannungsversorgungseinheit mit der ersten oder zweiten Elektrode über einen ersten Schalter zu steuern und die elektrische Verbindung der Messeinheit mit der ersten oder zweiten Elektrode über einen zweiten Schalter zu steuern, wobei die Messeinheit und der zweite Schalter parallel zu der Hochfrequenz-Hochspannungsversorgungseinheit und dem ersten Schalter in einem von dem Elektrochirurgiesystem gebildeten Stromkreis angeordnet sind. Durch eine solche parallele Anordnung der Stromkreise der Messeinheit und der Hochfrequenz-Hochspannungsversorgungseinheit des Elektrochirurgiesystems, kann sichergestellt werden, dass Gerätewiderstände der Messeinheit nicht die Behandlung des Gewebes durch das Elektrochirurgiesystem beeinflussen. Weiterhin wird durch die Nutzung des ersten und zweiten Schalters eine komplette elektronische Abkopplung eines Stromkreises der Messeinheit im Rahmen eines Messprozesses mit dem Stromkreis der Hochfrequenz-Hochspannungsversorgungseinheit während des Behandlungsprozesses ermöglicht.

In einer besonders bevorzugten Ausführungsform des Elektrochirurgiesystems sind der erste und zweite Schalter durch antiseriell zueinander verschaltete Feldeffekttransistoren gebildet. Die antiserielle Schaltung ermöglicht hierbei den ersten und den zweiten Schalter durch ein einziges Steuersignal zu steuern. Hierdurch kann das Steuern des ersten und zweiten Schalters zu dem Messbeginn und dem Messabschluss jeweils vorteilhaft aneinander gekoppelt werden. Die Nutzung von Feldeffekttransistoren ermöglicht weiterhin die Verwendung eines sehr geringen Schaltstroms, um den ersten und zweiten Schalter jeweils zu schalten. Weiterhin lässt sich eine Anordnung des ersten und zweiten Schalters gemäß der vorliegenden Ausführungsform besonders platzsparender und günstig realisieren.

In einer bevorzugten Ausführungsform ist die Messeinheit mit der Steuereinheit elektrisch verbunden und ausgebildet, abhängig von den bestimmten Eigenschaften des Gewebes einen Betriebszustand der Steuereinheit zu verändern. In einer Variante dieser Ausführungsform ist die Messeinheit ausgebildet, für den Fall, dass eine Differenz der Impedanzen bei verschiedenen Frequenzen unterhalb eines Grenzwertes liegt, einen Zeitabstand bis zum nächsten Messbeginn geringer festzulegen als er bis zum letzten Messbeginn ausgebildet war. Hierdurch kann bei starker Austrocknung des zu behandelnden Gewebes eine Intensität der Behandlung durch die Hochfrequenz-Hochspannungsversorgungseinheit durch häufigere Behandlungspausen im Rahmen von Messprozessen verringert werden. In einer weiteren Variante ist die Messeinheit ausgebildet, für den Fall, dass eine Differenz der Impedanzen bei verschiedenen Frequenzen unterhalb eines weiteren Grenzwertes liegt, ein Abschalten der Hochfrequenz-Hochspannungsversorgungseinheit durch die Steuereinheit auszulösen. Als Betriebszustand kann im Rahmen dieser Ausführungsform beispielsweise ein Zeitabstand zwischen aufeinanderfolgenden Messbeginn-Zeitpunkten oder eine Unterscheidung zwischen aktivem und inaktivem Elektrochirurgiesystem. Insbesondere kann die Steuereinheit ausgebildet sein, eine Notstopp-Funktion für das Elektrochirurgiesystem bereitzustellen. Ein Notstopp liegt dann vor, wenn die Hochfrequenz-Hochspannungsversorgungseinheit bei Vorliegen bestimmter Parameterbereiche für die Eigenschaft des zu behandelnden Gewebes abgeschaltet wird.

Erfindungsgemäß wird zur Lösung der vorgenannten Aufgabe auch ein Elektrochirurgiegenerator zum Behandeln eines Gewebes vorgeschlagen der eine Hochfrequenz-Hochspannungsversorgungseinheit, eine Messeinheit und eine Steuereinheit aufweist. Die Hochfrequenz-Hochspannungsversorgungseinheit ist mit einer ersten Elektrode und mit einer zweiten Elektrode elektrisch verbindbar ist und ist ausgebildet, diese Elektroden mit einer vorbestimmten Behandlungs-Wechselspannung zu versorgen. Die Messeinheit ist mit der ersten und zweiten Elektrode elektrisch verbindbar und weist ein Messspannungsversorgungsmodul auf, welches ausgebildet ist, MessWechselspannung mindestens einer ersten und einer zweiten Frequenz bereitzustellen, wobei die Messeinheit weiterhin ausgebildet ist, im Betrieb der Messeinheit über die erste und zweite Elektrode ein elektrisches Antwortsignal für die erste und zweite Frequenz aufzunehmen und daraus mindestens eine Eigenschaft eines zwischen der ersten und zweiten Elektrode befindlichen Gewebes zu bestimmen. Weiterhin ist die Steuereinheit ausgebildet, während eines Betriebs des Elektrochirurgiegenerators zu einem Messbeginn eine elektrische Verbindung der Hochfrequenz-Hochspannungsversorgungseinheit mit der ersten oder zweiten Elektrode zu unterbrechen und eine elektrische Verbindung der Messeinheit mit der ersten oder zweiten Elektrode sicherzustellen. Zusätzlich ist die Steuereinheit ausgebildet zu einem Messabschluss die elektrische Verbindung der Messeinheit mit der ersten oder zweiten Elektrode zu unterbrechen und die elektrische Verbindung der Hochfrequenz-Hochspannungsversorgungseinheit mit der ersten oder zweiten Elektrode sicherzustellen.

Der erfindungsgemäße Elektrochirurgiegenerator kann besonders vorteilhaft in einem einzigen Gehäuse bereitgestellt werden, welches mit der ersten und zweiten Elektrode über entsprechende Anschlüsse oder einen entsprechenden Anschluss verbindbar ist. Hierdurch kann vorteilhaft ein leichter Transport des Geräts und eine schnelle Signalübertragung zwischen Messeinheit, Steuereinheit und Hochfrequenz-Hochspannungsversorgungseinheit sichergestellt sein.

Zur Lösung der vorgenannten Aufgabe wird außerdem ein Verfahren zum Betreiben eines Elektrochirurgiesystems vorgeschlagen. Das offenbarte Verfahren weist die folgenden Verfahrensschritte auf:
- Auslösen eines Messbeginns des Elektrochirurgiesystems;
- Unterbrechen einer elektrischen Verbindung einer Hochspannungsversorgungseinheit mit einer ersten oder zweiten Elektrode;
- Anlegen einer von einer Messeinheit bereitgestellten Mess-Wechselspannung mit einer ersten Frequenz an die erste und zweite Elektrode;
- Aufnehmen eines elektronischen Antwortsignals für die erste Frequenz und Bestimmen einer Eigenschaft eines zwischen der ersten und zweiten Elektrode befindlichen Gewebes;
- Anlegen einer von der Messeinheit bereitgestellten Mess-Wechselspannung mit einer zweiten Frequenz an die erste und zweite Elektrode;
- Aufnehmen eines elektronischen Antwortsignals für die zweite Frequenz und Bestimmen der Eigenschaft des zwischen der ersten und zweiten Elektrode befindlichen Gewebes;
- Unterbrechen einer elektrischen Verbindung der Messeinheit mit der ersten oder zweiten Elektrode.
- Anlegen einer von der Hochfrequenz-Hochspannungsversorgungseinheit bereitgestellten Behandlungs-Wechselspannung an der ersten und zweiten Elektrode.

Vorteilhaft ist an dem Verfahren, dass es beliebig oft wiederholbar ist, um entsprechend oft ein Antwortsignal für die erste und zweite Frequenz aufzunehmen. Aus dem Antwortsignal kann entsprechend auf die Eigenschaft des zwischen den Elektroden befindlichen Gewebes geschlossen werden. Beispielsweise kann eine Impedanz des Gewebes bestimmt werden. In einer weiteren Variante kann eine Differenz der Impedanzen bei zwei verschiedenen Frequenzen genutzt werden, um als Maß für eine Austrocknung des Gewebes verwendet zu werden.

Vorteilhaft ist an dem Verfahren weiterhin, dass es mit einem Messbeginn startet, so dass sichergestellt werden kann, dass das zu behandelnde Gewebe die erwarteten elektrischen Parameter von unbehandeltem Gewebe aufweist. Hierdurch wird die Sicherheit des entsprechend betriebenen Elektrochirurgiesystems erhöht. Vorzugsweise wird der Schritt des Anlegens einer weiteren Frequenz durch die Messeinheit und das Aufnehmen eines elektronischen Antwortsignals für de weitere Frequenz sowie das Bestimmen der Eigenschaft des Gewebes für mehr als zwei Frequenzen, insbesondere für aus einem kontinuierlichen Frequenzspektrum auswählbare Frequenzen, ausgeführt.

In einer weiteren bevorzugten Ausführungsform geht dem Unterbrechen der elektrischen Verbindung mit der Messeinheit ein Auslösen eines Messabschlusses voraus. In dieser Ausführungsform ist eine Dauer des erfindungsgemäßen Verfahrens nicht vorbestimmt, sondern wird über ein externes Gerät, insbesondere über eine Nutzereingabe, ausgelöst. Hierdurch kann die Dauer der Messung der Messeinheit an eine vorliegende Behandlungssituation manuell angepasst werden. In einer alternativen Ausführungsform wird kein Messabschluss ausgelöst, sondern die Behandlung-Wechselspannung nach einer vordefinierten Messdauer wieder angelegt.

In einer besonders bevorzugten Ausführungsform erfolgt das Auslösen des Messbeginns des Elektrochirurgiesystems automatisiert in bestimmten, insbesondere vordefinierten Zeitabständen. Diese vordefinierten Zeitabstände können konstant, alternierend, wachsend oder sinkend sein. Insbesondere können sie abhängig von einen vor oder während der Behandlung bestimmten Hauttyp des zu behandelnden Gewebes sein. Solch ein automatisierter Messbeginn verringert vorteilhaft eine Anzahl von Aktionen, die ein Nutzer des entsprechenden Elektrochirurgiesystems ausführen muss.

In einer Ausführungsform des Verfahrens erfolgt das Anlegen einer jeweiligen Wechselspannung und das Unterbrechen einer elektrischen Verbindung jeweils über ein entsprechendes Schalten eines ersten Schalters, für die elektrische Verbindung der Hochfrequenz-Hochspannungsversorgungseinheit mit der ersten oder zweiten Elektrode, und eines zweiten Schalters, für die elektrischen Verbindung der Messeinheit mit der ersten oder zweiten Elektrode. In einer besonders bevorzugten Variante dieser Ausführungsform werden der erste Schalter und der zweite Schalter durch antiseriell zueinander verschaltete Feldeffekttransistoren gebildet.

Offenbart ist darüber hinaus das Verwenden des erfindungsgemäßen Elektrochirurgiesystems gemäß mindestens einer der beschriebenen Ausführungsformen zur Behandlung von Gewebe, insbesondere zur Koagulation oder zur Versieglung von Körpergewebe.

Offenbart ist auch das Anwenden des beschriebenen Verfahrens bei der Behandlung von Gewebe, insbesondere bei der Koagulation oder zur Versieglung von Körpergewebe.

Die Erfindung soll nun anhand von Ausführungsbeispielen mit Bezug auf die Figuren näher erläutert werden. Von den Figuren illustriert:
- Fig. 1: ein erstes Ausführungsbeispiel für ein erfindungsgemäßes Elektrochirurgiesystem;
- Fig. 2: ein zweites Ausführungsbeispiel für ein erfindungsgemäßes Elektrochirurgiesystem;
- Fig. 3: ein Ausführungsbeispiel für einen erfindungsgemäßen Elektrochirurgiegenerator;
- Fig.4: eine Illustration einer zeitlichen Abfolge von Behandlungszustand und Messzustand des erfindungsgemäßen Elektrochirurgiesystems;
- Fig. 5: ein Ausführungsbeispiel für ein Verfahren zum Betreiben eines Elektrochirurgiesystems.

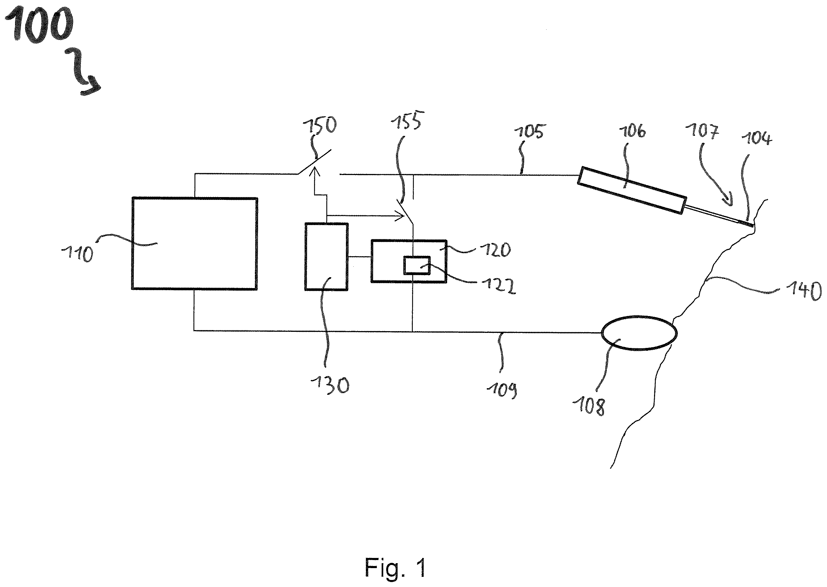

Fig. 1 zeigt ein erstes Ausführungsbeispielen für ein erfindungsgemäßes Elektrochirurgiesystem 100.

Das Elektrochirurgiesystem 100 hat eine erste Elektrode 104 und eine zweite Elektrode 108, eine Hochfrequenz-Hochspannungsversorgungseinheit 110, eine Messeinheit 120, und eine Steuereinheit 130.

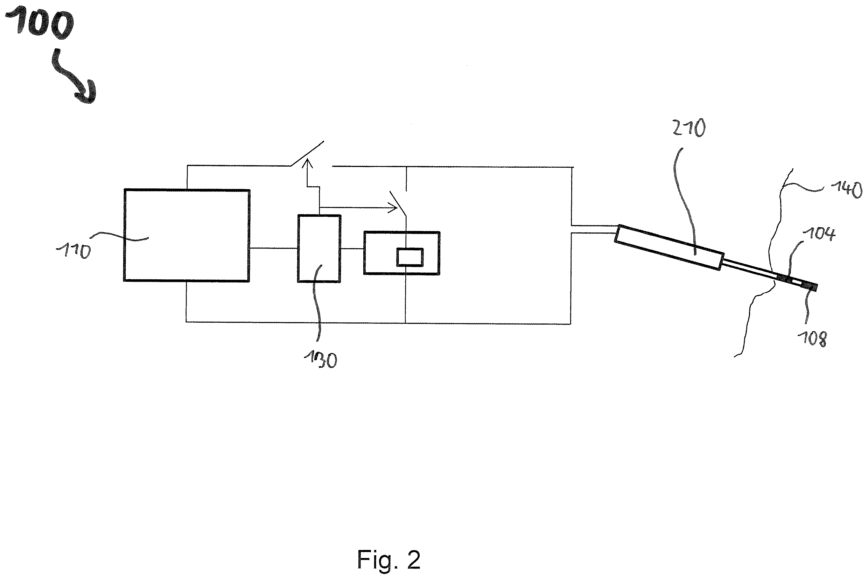

Die beiden Elektroden 104,108 sind leitend mit dem zu behandelnden Gewebe 140 verbunden. In dem gezeigten Ausführungsbeispiel ist die erste Elektrode 104 eine aktive Elektrode mit einem Griffbereich 106 und einem distalen Ende 107 und die zweite Elektrode 108 die entsprechende passive Elektrode. Ein Abstand der beiden Elektroden während einer Behandlung an dem Gewebe 110 ist derart gewählt, dass bei anliegender Wechselspannung ein Lichtbogen zwischen den beiden Elektroden 104, 108 entstehen kann. In dem in Fig. 2 dargestellten Ausführungsbeispiel liegen die erste und zweite Elektrode in einem gemeinsamen Elektrochirurgie-Instrument vor.

Die Hochfrequenz-Hochspannungsversorgungseinheit 110 ist mit der ersten Elektrode 104 über eine erste elektrische Leitung 105 und mit der zweiten Elektrode 108 über eine zweite elektrische Leitung 109 elektrisch verbindbar. Weiterhin ist die Hochfrequenz-Hochspannungsversorgungseinheit 110 ausgebildet, die beiden Elektroden 104, 108 mit einer vorbestimmten Behandlungs-Wechselspannung zu versorgen. Die Wechselspannung liegt in dem dargestellten Ausführungsbeispiel zwischen 350 kHz und 500 kHz.

Die Messeinheit 120 ist ebenfalls mit der ersten und zweiten Elektrode 104,108 elektrisch verbindbar. Weiterhin weist die Messeinheit 120 ein Messspannungsversorgungsmodul 122 auf, welches ausgebildet ist, Mess-Wechselspannung mindestens einer ersten und zweiten Frequenz bereitzustellen. Weiterhin ist die Messeinheit 120 ausgebildet, über die erste und zweite Elektrode 104,108 ein elektrisches Antwortsignal für die erste und zweite Frequenz aufzunehmen und daraus mindestens eine Eigenschaft des zwischen der ersten und zweiten Elektrode 104,108 befindlichen Gewebes 140 zu bestimmen.

Die Steuereinheit 130 ist ausgebildet, während eines Betriebs des Elektrochirurgiesystems 100 zu einem Messbeginn die elektrische Verbindung der Hochfrequenz-Hochspannungsversorgungseinheit 110 mit der ersten oder zweiten Elektrode 104, 108 zu unterbrechen und die elektrische Verbindung der Messeinheit 120 mit der ersten und zweiten Elektrode 104, 108 sicherzustellen. Zu einem Messabschluss ist die Steuereinheit 130 weiterhin ausgebildet, die elektrische Verbindung der Messeinheit 120 mit der ersten oder zweiten Elektrode 104, 108 zu unterbrechen und die elektrische Verbindung der Hochfrequenz-Hochspannungsversorgungseinheit 110 mit der ersten und zweiten Elektrode 104, 108 sicherzustellen.

Weiterhin illustriert Fig. 1 einen ersten Schalter 150, durch den die Hochfrequenz-Hochspannungsversorgungseinheit 110 von der ersten Elektrode 104 getrennt werden kann. Parallel zu der Hochfrequenz-Hochspannungsversorgungseinheit 110 und dem ersten Schalter 150 sind die Messeinheit 120 und ein zweiter Schalter 155 geschaltet. Durch den zweiten Schalter 155 kann die Messeinheit 120 mit der ersten Elektrode 104 elektrisch verbunden werden oder es kann eine vorliegende elektrische Verbindung zwischen Messeinheit 120 und der ersten Elektrode 104 unterbrochen werden. Vorliegend sind der erste und zweite Schalter 150,155 als antiseriell zueinander verschaltet Feldeffekttransistoren ausgebildet (aus Gründen der Übersichtlichkeit ist dies nicht dargestellt).

Die Steuereinheit 130 ist in dem dargestellten Ausführungsbeispiel zusätzlich ausgebildet, den Messbeginn automatisiert in vordefinierten Zeitabständen auszuführen, wobei eine zwischen Messbeginn und Messabschluss durchgeführte Messung der Messeinheit 120 eine vordefinierte Messdauer aufweist. In dem vorliegenden Elektrochirurgiesystem 100 wechseln sich der Behandlungsprozess mit der Behandlungs-Wechselspannung und der Messprozess mit an den Elektroden 104,108 anliegender Mess-Wechselspannung daher in vordefinierten Zeitabständen ab. Die vordefinierten Zeitabstände sind in dem vorliegenden Ausführungsbeispiel konstant. In anderen nicht dargestellten Ausführungsbeispielen sinken die vordefinierten Zeitabstände in vordefinierter Weise exponentiell mit der Zeit.

Das vorliegende Elektrochirurgiesystem 100 ermöglicht eine regelmäßiger und automatisierte Kontrolle von Eigenschaften des zu behandelnden Gewebes 140. Weiterhin wird durch eine räumliche und zeitliche Trennung zwischen Behandlungsprozess und Messprozess sichergestellt, dass sich beide Prozesse nicht beeinflussen.

Aus Gründen der Übersichtlichkeit ist in Fig. 1 nicht dargestellt, dass die Messeinheit 120 ein Signalverarbeitungsmodul aufweist, welches die elektrischen Antwortsignale für die erste und zweite Frequenz empfängt. Das Signalverarbeitungsmodul ist dabei ausgebildet, die Eigenschaft des Gewebes 140 auf Basis eines Vergleichs mit einem vorbestimmten und dem Signalverarbeitungsmodul bereitgestellten Wert zu bestimmen. Weiterhin ist die vorliegende Messeinheit 120 ausgebildet, ein Informationssignal auf Basis der bestimmten Eigenschaft des Gewebes 140 über eine Nutzerschnittstelle durch eine optische Ausgabe, beispielsweise über ein Display des Elektrochirurgiesystems 100, auszugeben.

In einem nicht dargestellten Ausführungsbeispielen ist die Steuereinheit mit einer Nutzerschnittstelle verbunden und weiterhin ausgebildet, den Messbeginn bei Vorliegen einer manuellen Nutzereingabe, beispielsweise durch Drücken einer Taste, auszulösen.

Fig. 2 zeigt ein zweites Ausführungsbeispiel für ein erfindungsgemäßes Elektrochirurgiesystem 100.

Das Elektrochirurgiesystem 100 gemäß dem dargestellten zweiten Ausführungsbeispiel unterscheidet sich von dem in Fig. 1 gezeigten Ausführungsbeispiel dadurch, dass die erste Elektrode 104 und die zweite Elektrode 108 an einem distalen Ende eines gemeinsamen Elektrochirurgieinstruments 210 angeordnet sind. Hierbei ist ein räumlicher Abstand zwischen der ersten Elektrode 104 und der zweiten Elektrode 108 fixiert. Eine genaue Lage von elektrischen Verbindungskabeln innerhalb des Elektrochirurgieinstruments 210 ist aus Gründen der Übersichtlichkeit in Fig. 2 nicht dargestellt, jedoch wohlbekannt im Bereich der HF-Chirurgie.

Weiterhin unterscheidet sich das in Fig. 2 dargestellte Elektrochirurgieinstrument 100 von demjenigen aus Fig. 1 dadurch, dass die Steuereinheit 130 mit der Hochfrequenz-Hochspannungsversorgungseinheit 110 elektrisch verbunden ist. Die Steuereinheit 130 ist hierbei zusätzlich ausgebildet, einen Betriebszustand der Hochfrequenz-Hochspannungsversorgungseinheit 110 abhängig von der durch die Messeinheit bestimmten Eigenschaft des zu behandelnden Gewebes 140 zu verändern. Insbesondere wird die Hochfrequenz-Hochspannungsversorgungseinheit 110 durch die Steuereinheit 130 abgeschaltet, falls die am Gewebe 140 gemessene Impedanz einen vordefinierten Schwellwert unterschreitet, also wenn das Gewebe 140 beispielsweise einen kritischen Grad an Carbonisierung erreicht hat.

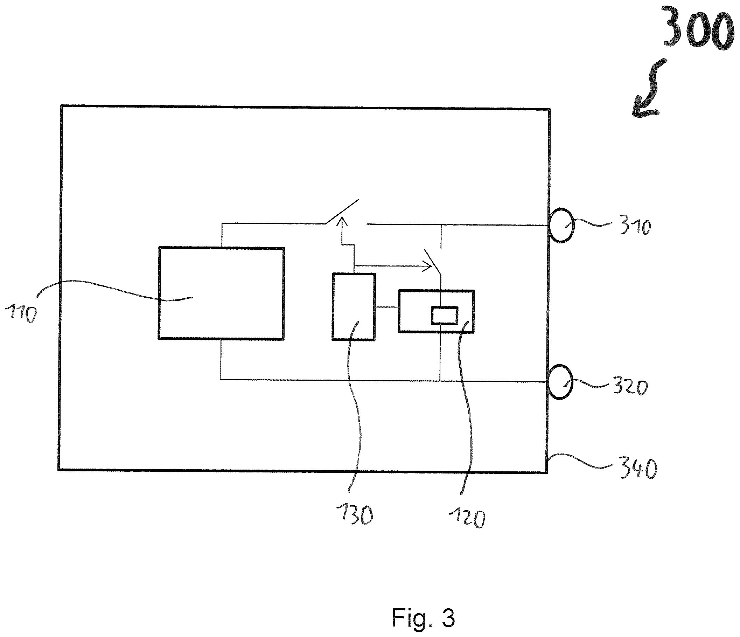

Fig. 3 zeigt ein Ausführungsbeispiel für einen erfindungsgemäßen Elektrochirurgiegenerator 300.

Der dargestellte Elektrochirurgiegenerator 300 weist den in Fig. 1 dargestellten Aufbau und die entsprechende Anordnung von Hochfrequenzhochspannungseinheit 110, Messeinheit 120 und Steuereinheit 130 auf. Elektroden sind an den dargestellten Elektrochirurgiegenerator 300 über einen ersten Anschluss 310 und einen zweiten Anschluss 320 anschließbar. Der Elektrochirurgiegenerator 300 weist weiterhin ein gemeinsames Gehäuse 340 auf, in welchem der erste Anschluss 310 und der zweite Anschluss 320 ausgebildet sind, und welches die Hochfrequenz-Hochspannungsversorgungseinheit 110, die Messeinheit 120 und die Steuereinheit 130 umfasst.

Der Elektrochirurgiegenerator 300 ist dabei vorteilhaft derart ausgebildet, dass handelsübliche Elektroden zur Behandlung von Gewebe an den ersten Anschluss 310 und an den zweiten Anschluss 320 angeschlossen werden können.

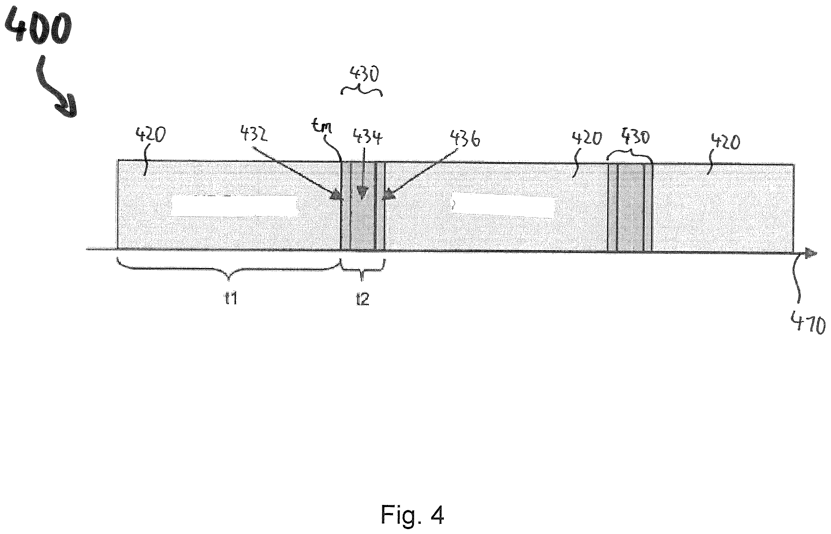

Fig. 4 zeigt eine Illustration einer zeitlichen Abfolge 400 von Behandlungsprozess 420 und Messprozess 430 des erfindungsgemäßen Elektrochirurgiesystems.

Die zeitliche Abfolge 400 ist entlang einer Zeitachse 410 dargestellt. In dem dargestellten Ausführungsbeispiel ist eine Behandlungsdauer t1 eines Behandlungsprozesses 420 konstant, ändert sich also nicht mit der Zeit. Der Behandlungsprozess 420 wird regelmäßig wiederholt, wobei zwischen zwei Phasen des Behandlungsprozesses 420 stets ein Messprozess 430 mit einer konstant gehaltenen Messdauer t2 ausgeführt wird. Eine Behandlung des Gewebes besteht dabei aus einer Vielzahl von Behandlungsprozessen 420.

Der Wechsel zwischen Behandlungsprozess 420 und Messprozess 430 wird durch den Zeitpunkt tm definiert, an dem der Messprozess 430 ausgelöst wird. Der Messprozess besteht in dem vorliegenden Ausführungsbeispiel aus 3 Phasen, die durch Messbeginn 432, Messung 434 und Messabschluss 436 gebildet sind. Während des Messbeginns 432 wird die elektrische Verbindung der Elektroden zur Hochfrequenz-Hochspannungsversorgungseinheit unterbrochen und die elektrische Verbindung zwischen Messeinheit und den beiden Elektroden wird sichergestellt. Die Messung 434 beginnt mit einem Aktivieren der Messwechselspannung für eine erste Frequenz. Die Messung 434 endet mit einem Abschalten der Messwechselspannung. Während des Messabschlusses 436 wird die elektrische Verbindung zwischen Messeinheit und den beiden Elektroden unterbrochen und die elektrische Verbindung zwischen den Elektroden und der Hochfrequenz-Hochspannungsversorgungseinheit wird sichergestellt.

Das Auslösen des Messprozesses 430 zum jeweiligen Zeitpunkt tm erfolgt automatisiert in regelmäßigen Zeitintervallen der Dauer (t1+t2).

In nicht dargestellten Ausführungsbeispielen sind Messbeginn, Messung und Messabschluss nicht klar voneinander getrennt, da eine von der Messeinheit bereitgestellte Messwechselspannung über einen kompletten Zeitraum der Behandlung vorliegt, so das nach dem Bereitstellen einer entsprechenden elektrischen Verbindung ein Messvorgang an dem Gewebe beginnt. In weiteren nicht dargestellten Ausführungsbeispielen sind die Behandlungsdauer und die Messdauer nicht über einen kompletten Zeitraum der Behandlung des Gewebes konstant. Insbesondere ist die Behandlungsdauer zwischen zwei Messprozessen in einer Variante des Elektrochirurgiesystems über den Zeitraum der Behandlung sinkend, beispielsweise exponentiell sinkend.

In einem nicht dargestellten Ausführungsbeispiel beträgt die Behandlungsdauer t1 100 ms. In einem weiteren nicht dargestellten Ausführungsbeispiel beträgt die Messdauer t2 weniger als 100 ms.

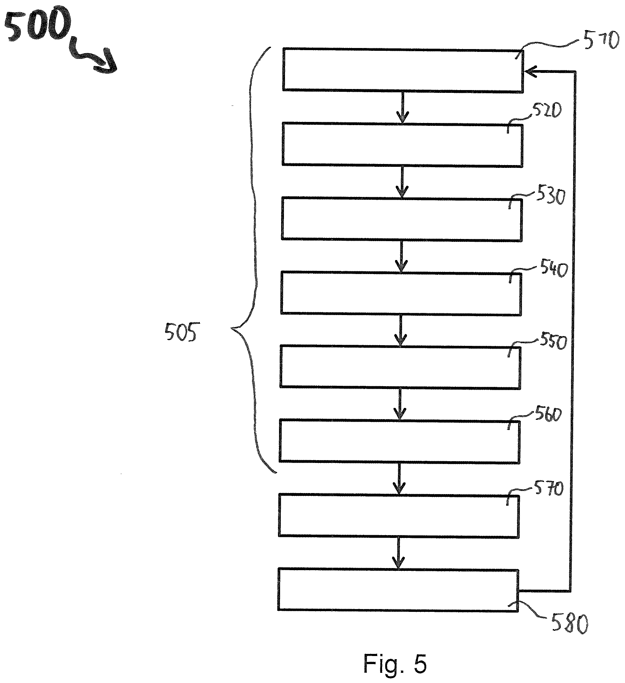

Fig. 5 zeigt ein Verfahren 500 zum Betreiben eines Elektrochirurgiesystems, mit den im Folgenden erläuterten Verfahrensschritten.

Ein erster Schritt 510 wird ein Messbeginn des Elektrochirurgiesystems ausgelöst.

Daraufhin erfolgt in einem weiteren Schritt 520 ein Unterbrechen einer elektrischen Verbindung der Hochfrequenz-Hochspannungsversorgungseinheit mit der ersten oder zweiten Elektrode.

Hierauf folgt ein Anlegen einer von einer Messeinheit bereitgestellten MessWechselspannung mit einer ersten Frequenz an die erste und zweite Elektrode in einem weiteren Schritt 530.

In einem nächsten Schritt 540 folgt ein Aufnehmen eines elektronischen Antwortsignals für die erste Frequenz und ein Bestimmen einer Eigenschaft eines zwischen der ersten und zweiten Elektrode befindlichen Gewebes.

Hieraufhin folgt der nächste Schritt 550, in dem Schritt 530 für eine zweite Frequenz wiederholt wird.

Erneut wird hierbei ein elektrisches Antwortsignals aufgenommen. In diesem Schritt 560 wird wiederum aus dem elektrischen Antwortsignal für die zweite Frequenz auf die Eigenschaft des zwischen der ersten und zweiten Elektrode befindlichen Gewebes geschlossen.

Zum Abschluss eines durch die Schritte 510, 520, 530, 540, 550, 560 durchgeführten Messprozesses 505 wird im Schritt 570 einer elektrischen Verbindung der Messeinheit mit der ersten oder zweiten Elektrode unterbrochen.

Abschließend umfasst der Schritt 580 ein Anlegen einer von der Hochfrequenz-Hochspannungsversorgungseinheit bereitgestellten Behandlungs-Wechselspannung an der ersten und zweiten Elektrode.

Der erste Schritt 510 wiederholt sich in dem vorliegenden Ausführungsbeispiel regelmäßig. So wiederholen sich die dargestellten Schritte 510, 520, 530, 540, 550, 560, 570, 580 in der illustrierten Reihenfolge bis zu einem Ende der Behandlung des Gewebes.

In nicht dargestellten Ausführungsbeispielen erfolgen nach Schritt 560 und vor Schritt 570 zusätzlich das Anlegen einer Mess-Wechselspannung mindestens einer weiteren Frequenz und ein entsprechendes Aufnehmen des elektrischen Antwortsignals und ein entsprechendes Bestimmen einer Eigenschaft des zu behandelnden Gewebes.

Vorliegend handelt es sich bei der Eigenschaft um die Impedanz des Gewebes, jeweils bei unterschiedlichen Frequenzen der angelegten Mess-Wechselspannung. In nicht dargestellten Ausführungsbeispielen umfasst das Bestimmen der Eigenschaft noch ein Bestimmen einer Differenz von Impedanzen, zwischen Impedanzen verschiedenen Frequenzen der Mess-Wechselspannung.

### Bezuqszeichenliste:

- 100: Elektrochirurgiesystem
- 104: erste Elektrode
- 105: erste elektrische Leitung
- 106: Griffbereich
- 107: distales Ende
- 108: zweite Elektrode
- 109: zweite elektrische Leitung
- 110: Hochfrequenz-Hochspannungsversorgungseinheit
- 120: Messeinheit
- 122: Messspannungsversorgungsmodul
- 130: Steuereinheit
- 140: Gewebe
- 150: erster Schalter
- 155: zweiter Schalter
- 210: Elektrochirurgieinstrument
- 300: Elektrochirurgiegenerator
- 310: erster Anschluss
- 320: zweiter Anschluss
- 340: Gehäuse
- 400: zeitliche Abfolge
- 410: Zeitachse
- 420: Behandlungsprozess
- 430,505: Messprozess
- 432: Messbeginn
- 434: Messung
- 436: Messabschluss
- 500: Verfahren
- 510: erster Verfahrensschritt
- 520, 530, 540, 550, 560, 570: Verfahrensschritte des Messprozesses innerhalb des Verfahrens
- 580: Abschluss des Messprozesses innerhalb des Verfahrens
- t1: Behandlungsdauer
- t2: Messdauer
- tm: Wechsel von Behandlungsprozess zu Messprozess

## Patentansprüche

1. Elektrochirurgiesystem zum Behandeln eines Gewebes, mit
- einer ersten Elektrode und einer zweiten Elektrode, die jeweils ausgebildet sind, leitend mit dem zu behandelnden Gewebe verbunden zu sein;
- einer Hochfrequenz-Hochspannungsversorgungseinheit, die mit der ersten Elektrode über eine erste elektrische Leitung und mit der zweiten Elektrode über eine zweite elektrische Leitung elektrisch verbindbar ist und die ausgebildet ist, diese Elektroden mit einer vorbestimmten Behandlungs-Wechselspannung zu versorgen;
- einer Messeinheit, die mit der ersten und zweiten Elektrode elektrisch verbindbar ist und die ein Messspannungsversorgungsmodul aufweist, welches ausgebildet ist, Mess-Wechselspannung mindestens einer ersten und einer zweiten Frequenz bereitzustellen, wobei die Messeinheit weiterhin ausgebildet ist, über die erste und zweite Elektrode ein elektrisches Antwortsignal für die erste und zweite Frequenz aufzunehmen und daraus mindestens eine Eigenschaft eines zwischen der ersten und zweiten Elektrode befindlichen Gewebes zu bestimmen;
- einer Steuereinheit, die ausgebildet ist, während eines Betriebs des Elektrochirurgiesystems
zu einem Messbeginn die elektrische Verbindung der Hochfrequenz-Hochspannungsversorgungseinheit mit der ersten oder zweiten Elektrode zu unterbrechen und die elektrische Verbindung der Messeinheit mit der ersten und zweiten Elektrode sicherzustellen, und
zu einem Messabschluss die elektrische Verbindung der Messeinheit mit der ersten oder zweiten Elektrode zu unterbrechen und die elektrische Verbindung der Hochfrequenz-Hochspannungsversorgungseinheit mit der ersten und zweiten Elektrode sicherzustellen,
wobei die Steuereinheit mit der Messeinheit elektrisch verbunden ist ,
**dadurch gekennzeichnet, dass** die Steuereinheit weiterhin ausgebildet ist, nach dem Messbeginn eine Aktivierung der Mess-Wechselspannung mit der ersten Frequenz durch das Messspannungsversorgungsmodul auszulösen und vor dem Messabschluss eine Deaktivierung der Mess-Wechselspannung des Messspannungsversorgungsmoduls auszulösen.

2. Elektrochirurgiesystem gemäß Anspruch 1, bei dem die Messeinheit ausgebildet ist, die Impedanz des zwischen der ersten und zweiten Elektrode befindlichen Gewebes jeweils für die Mess-Wechselspannung mit der ersten und zweiten Frequenz zu bestimmen.

3. Elektrochirurgiesystem gemäß mindestens einem der vorhergehenden Ansprüche, bei dem eine Zeitdauer zwischen Messbeginn und Messabschluss eine vordefinierte Messdauer ist.

4. Elektrochirurgiesystem gemäß Anspruch 3, welches derart konfiguriert ist, dass der Messbeginn automatisiert in bestimmten Zeitabständen von der Steuereinheit ausgelöst wird.

5. Elektrochirurgiesystem gemäß mindestens einem der vorhergehenden Ansprüche, bei dem die Steuereinheit ausgebildet ist, die elektrische Verbindung der Hochfrequenz-Hochspannungsversorgungseinheit mit der ersten oder zweiten Elektrode über einen ersten Schalter zu steuern und die elektrische Verbindung der Messeinheit mit der ersten oder zweiten Elektrode über einen zweiten Schalter zu steuern, wobei die Messeinheit und der zweite Schalter parallel zu der Hochfrequenz-Hochspannungsversorgungseinheit und dem ersten Schalter in einem von dem Elektrochirurgiesystem gebildeten Stromkreis angeordnet sind.

6. Elektrochirurgiesystem gemäß Anspruch 5, bei dem der erste und zweite Schalter durch antiseriell zueinander verschaltete Feldeffekttransistoren gebildet sind.

7. Elektrochirurgiesystem gemäß mindestens einem der vorhergehenden Ansprüche, bei dem die Messeinheit mit der Steuereinheit elektrisch verbunden ist und ausge bildet ist, abhängig von den bestimmten Eigenschaften des Gewebes einen Betriebszustand der Steuereinheit zu verändern.

8. Elektrochirurgiegenerator zum Behandeln eines Gewebes, mit einer Hochfrequenz-Hochspannungsversorgungseinheit, die mit einer ersten Elektrode und mit einer zweiten Elektrode elektrisch verbindbar ist und die ausgebildet ist, diese Elektroden mit einer vorbestimmten Behandlungs-Wechselspannung zu versorgen;
- einer Messeinheit, die mit der ersten und zweiten Elektrode elektrisch verbindbar ist und die ein Messspannungsversorgungsmodul aufweist, welches ausgebildet ist, Mess-Wechselspannung mindestens einer ersten und einer zweiten Frequenz bereitzustellen, wobei die Messeinheit weiterhin ausgebildet ist, im Betrieb der Messeinheit über die erste und zweite Elektrode ein elektrisches Antwortsignal für die erste und zweite Frequenz aufzunehmen und daraus mindestens eine Eigenschaft eines zwischen der ersten und zweiten Elektrode befindlichen Gewebes zu bestimmen;
- einer Steuereinheit, die ausgebildet ist, während eines Betriebs des Elektrochirurgiegenerators
zu einem Messbeginn eine elektrische Verbindung der Hochfrequenz-Hochspannungsversorgungseinheit mit der ersten oder zweiten Elektrode zu unterbrechen und eine elektrische Verbindung der Messeinheit mit der ersten oder zweiten Elektrode sicherzustellen, und
zu einem Messabschluss die elektrische Verbindung der Messeinheit mit der ersten oder zweiten Elektrode zu unterbrechen und die elektrische Verbindung der Hochfrequenz-Hochspannungsversorgungseinheit mit der ersten oder zweiten Elektrode sicherzustellen,
wobei die Steuereinheit mit der Messeinheit elektrisch verbunden ist ,**dadurch gekennzeichnet, dass** die Steuereinheit
c)- weiterhin ausgebildet ist, nach dem Messbeginn eine Aktivierung der Mess-Wechselspannung mit der ersten Frequenz durch das Messspannungsversorgungsmodul auszulösen und vor dem Messabschluss eine Deaktivierung der Mess-Wechselspannung des Messspannungsversorgungsmoduls auszulösen.

## Claims

1. Electrosurgical system for treating a tissue, comprising:
- a first electrode and a second electrode, each configured to be conductively connected to the tissue to be treated;
- a high-frequency high-voltage supply unit that can be electrically connected to the first electrode via a first electrical line and to the second electrode via a second electrical line, and that is configured to supply a specified AC treatment voltage to these electrodes;
- A measuring unit that can be electrically connected to the first electrode and second electrode and that has a measurement voltage supply module that is configured to supply an AC measurement voltage of at least a first frequency and a second frequency, wherein the measuring unit is further configured to receive, via the first electrode and second electrode, an electrical response signal for the first frequency and second frequency and to use it to determine at least one property of a tissue located between the first electrode and second electrode;
- a control unit that is configured, during operation of the electrosurgical system,
at the start of a measurement, to interrupt the electrical connection of the high-frequency high-voltage supply unit to the first or second electrode and ensure the electrical connection of the measuring unit to the first electrode and second electrode; and,
at the end of a measurement, to interrupt the electrical connection of the measuring unit to the first or second electrode and ensure the electrical connection of the high-frequency high-voltage supply unit to the first electrode and second electrode,
wherein the control unit is electrically connected to the measuring unit, **characterized in that** the control unit is further configured to trigger, after the start of a measurement, an activation of the AC measurement voltage at the first frequency via the measurement voltage supply module and to trigger, before the end of the measurement, a deactivation of the AC measurement voltage of the measurement voltage supply module.

2. Electrosurgical system according to claim 1, wherein the measuring unit is configured to determine the impedance of the tissue located between the first electrode and second electrode for the AC measurement voltage at the first frequency and second frequency, respectively.

3. Electrosurgical system according to at least one of the preceding claims, in which a time interval between the start of the measurement and the end of the measurement is a specified measuring time.

4. Electrosurgical system according to claim 3, which is configured in such a way that the control unit automatically triggers the start of the measurement at specified time intervals.

5. Electrosurgical system according to at least one of the preceding claims, in which the control unit is configured to control the electrical connection of the high-frequency high-voltage supply unit to the first or second electrode via a first switch and the electrical connection of the measuring unit to the first or second electrode via a second switch, wherein the measuring unit and the second switch are arranged in parallel to the high-frequency high-voltage supply unit and to the first switch in an electric circuit formed by the electrosurgical system.

6. Electrosurgical system according to claim 5, in which the first switch and second switch are formed by field effect transistors interconnected by way of an antiserial connection.

7. Electrosurgical system according to at least one of the preceding claims, in which the measuring unit is electrically connected to the control unit and configured to change an operating mode of the control unit depending on the specific properties of the tissue.

8. Electrosurgical generator for treating a tissue, with a high-frequency high-voltage supply unit that can be electrically connected to a first electrode and to a second electrode, and that is configured to supply a specified AC treatment voltage to these electrodes;
- a measuring unit that can be electrically connected to the first electrode and second electrode and that has a measurement voltage supply module that is configured to supply an AC measurement voltage of at least a first frequency and a second frequency, wherein the measuring unit is further configured to receive, when the measuring unit is in operation, via the first electrode and second electrode, an electrical response signal for the first frequency and second frequency and to use it to determine at least one property of a tissue located between the first electrode and second electrode;
- a control unit that is configured, during operation of the electrosurgical generator,
at the start of a measurement, to interrupt an electrical connection of the high-frequency high-voltage supply unit to the first or second electrode and ensure an electrical connection of the measuring unit to the first or second electrode; and,
at the end of a measurement, to interrupt the electrical connection of the measuring unit to the first or second electrode and ensure the electrical connection of the high-frequency high-voltage supply unit to the first or second electrode,
wherein the control unit is electrically connected to the measuring unit, **characterized in that** the control unit is further configured to trigger, after the start of the measurement, an activation of the AC measurement voltage at the first frequency via the measurement voltage supply module and to trigger, before the end of the measurement, a deactivation of the AC measurement voltage of the measurement voltage supply module.

## Revendications

1. Système d'électrochirurgie pour le traitement d'un tissu, avec
- une première électrode et une deuxième électrode, qui sont respectivement réalisées pour être reliées de manière conductrice au tissu à traiter ;
- une unité d'alimentation haute tension haute fréquence, qui peut être reliée électriquement à la première électrode par l'intermédiaire d'une première ligne électrique et à la deuxième électrode par l'intermédiaire d'une deuxième ligne électrique et qui est réalisée pour alimenter ces électrodes avec une tension alternative de traitement prédéfinie ;
- une unité de mesure, qui peut être reliée électriquement à la première et deuxième électrode et qui présente un module d'alimentation en tension de mesure, lequel est réalisé pour fournir une tension alternative de mesure au moins d'une première et d'une deuxième fréquence, dans lequel l'unité de mesure est réalisée en outre pour recevoir un signal de réponse électrique pour la première et deuxième fréquence par l'intermédiaire de la première et deuxième électrode et pour déterminer à partir de celui-ci au moins une propriété d'un tissu se trouvant entre la première et deuxième électrode ;
- une unité de commande, qui est réalisée pendant un fonctionnement du système d'électrochirurgie à un début de mesure pour interrompre la liaison électrique de l'unité d'alimentation haute tension haute fréquence avec la première ou deuxième électrode et pour assurer la liaison électrique de l'unité de mesure avec la première et deuxième électrode, et
à une fin de mesure pour interrompre la liaison électrique de l'unité de mesure avec la première ou deuxième électrode et pour assurer la liaison électrique de l'unité d'alimentation haute tension haute fréquence avec la première et deuxième électrode,
dans lequel l'unité de commande est reliée électriquement à l'unité de mesure,
**caractérisé en ce que** l'unité de commande est réalisée en outre, après le début de mesure, pour déclencher une activation de la tension alternative de mesure avec la première fréquence au moyen du module d'alimentation en tension de mesure et avant la fin de mesure pour déclencher une désactivation de la tension alternative de mesure du module d'alimentation en tension de mesure.

2. Système d'électrochirurgie selon la revendication 1, pour lequel l'unité de mesure est réalisée pour déterminer l'impédance du tissu se trouvant entre la première et deuxième électrode respectivement pour la tension alternative de mesure avec la première et deuxième fréquence.

3. Système d'électrochirurgie selon au moins l'une des revendications précédentes, pour lequel une durée entre le début de mesure et la fin de mesure est une durée de mesure prédéfinie.

4. Système d'électrochirurgie selon la revendication 3, lequel est configuré de telle sorte que le début de mesure est déclenché de manière automatisée par intervalles de temps définies par l'unité de commande.

5. Système d'électrochirurgie selon au moins l'une des revendications précédentes, pour lequel l'unité de commande est réalisée pour commander la liaison électrique de l'unité d'alimentation haute tension haute fréquence avec la première ou deuxième électrode par l'intermédiaire d'un premier commutateur et pour commander la liaison électrique de l'unité de mesure avec la première ou deuxième électrode par l'intermédiaire d'un deuxième commutateur, dans lequel l'unité de mesure et le deuxième commutateur sont disposés parallèlement à l'unité d'alimentation haute tension haute fréquence et au premier commutateur dans un circuit formé par le système d'électrochirurgie.

6. Système d'électrochirurgie selon la revendication 5, pour lequel le premier et deuxième commutateur sont formés par des transistors à effet de champ branchés les uns aux autres en tête-bêche.

7. Système d'électrochirurgie selon au moins l'une des revendications précédentes, pour lequel l'unité de mesure est reliée électriquement à l'unité de commande et est réalisée pour modifier un état de fonctionnement de l'unité de commande en fonction des propriétés définies.

8. Générateur d'électrochirurgie pour le traitement d'un tissu, avec une unité d'alimentation haute tension haute fréquence, qui peut être reliée électriquement à une première électrode et à une deuxième électrode et qui est réalisée pour alimenter ces électrodes avec une tension alternative de traitement prédéfinie ;
- une unité de mesure, qui peut être reliée à la première et deuxième électrode et qui présente un module d'alimentation en tension de mesure, lequel est réalisé pour fournir une tension alternative de mesure au moins d'une première et d'une deuxième fréquence, dans lequel l'unité de mesure est réalisée en outre pour recevoir un signal de réponse électrique pour la première et deuxième fréquence lors du fonctionnement de l'unité de mesure et pour déterminer à partir de celui-ci au moins une propriété d'un tissu se trouvant entre la première et deuxième électrode ;
- une unité de commande, qui est réalisée pendant un fonctionnement du générateur d'électrochirurgie à un début de mesure pour interrompre une liaison électrique de l'unité d'alimentation haute tension haute fréquence avec la première ou deuxième électrode et pour assurer une liaison électrique de l'unité de mesure avec la première ou deuxième électrode, et
à une fin de mesure pour interrompre la liaison électrique de l'unité de mesure avec la première ou deuxième électrode et pour assurer la liaison électrique de l'unité d'alimentation haute tension haute fréquence avec la première ou deuxième électrode,
dans lequel l'unité de commande est reliée électriquement à l'unité de mesure, **caractérisé en ce que** l'unité de commande est réalisée en outre pour déclencher après le début de mesure une activation de la tension alternative de mesure avec la première fréquence au moyen du module d'alimentation en tension de mesure et pour déclencher avant la fin de mesure une désactivation de la tension alternative de mesure du module d'alimentation en tension de mesure.
